# EUROPEAN PATENT APPLICATION

(11) **EP 0 764 442 A1**
(43) Date of publication of application: **26.03.1997**
(21) Application number: 96306620.4
(22) Date of filing: 12.09.1996
(51) Int. Cl.: A61K 31/195

(54) **Use of specific sulphur containing aminos acid derivatives against pathogenic viral or prion particles**

(30) Priority: 19.09.1995 EP 95306587; 19.09.1995 US 530745; 19.09.1995 AU 31769/95; 19.02.1996 GB 9603471
(71) Applicant: Holt, John Alfred Gorton, Perth, Western Australia 6009 (AU)
(72) Inventor: Holt, John Alfred Gorton, Perth, Western Australia 6009 (AU)
(74) Representative: Brown, David Leslie

(57) **Abstract**

A therapeutic method for reducing the concentration of pathogenic viral and/or prion particles in body tissue and fluids, applicable *in vivo* to a patient or *in vitro* to a transfusable body fluid or transplantable body part, comprises administering to the patient, body fluid or body part an effective amount of a non-toxic active agent selected from cystine, oxidised glutathione, lower alkyl/alkenyl cysteine sulfoxides, and salts thereof, and while the non-toxic agent is present administering to the patient, body fluid or body part an effective dose of microwave electromagnetic radiation of frequency in the range of about 400-450 MHz.

The method is effective against infective particulate agents such as human immunodeficiency virus (HIV), bovine spongiform encephalopathy (BSE) agent, scrapie agent, hepatitis agent, disseminated sclerosis (MS) agent and Creutzfeld Jacob disease (CJD) agent.

## Description

The present invention relates to therapeutic methods and compositions for use therein.

European Patent Application No. 0531031 (the disclosure of which is incorporated herein by reference) describes a cancer therapy, and compositions for use therein, in which an effective amount of a non-toxic organic disulfide is administered to a patient and while said disulfide is present at a cancer site of the patient there is administered to the cancer site an effective dose of microwave electromagnetic radiation of frequency in the range of about 400-450 MHz.

European Patent Application No. 0616803 (the disclosure of which is incorporated herein by reference) describes the use of such methods in enhancing blood T-cell count in immunodeficient (e.g. HIV-positive) individuals.

In both documents, cystine, which has the formula 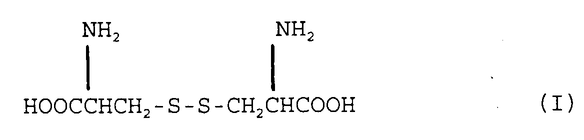 and non-toxic salts thereof are stated to be preferred disulfide agents.

Oxidised glutathione 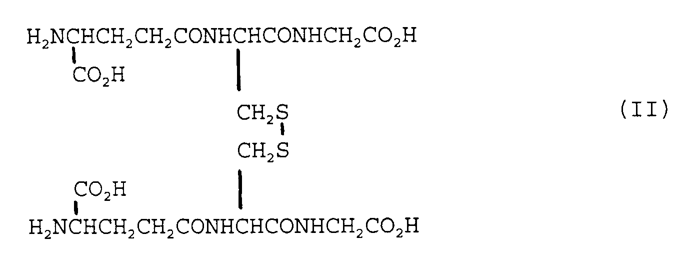 and non-toxic salts thereof are stated in EP-A-0616803 to be alternative preferred disulfide agents in the context of enhancement of a patient's T-cell count.

T-butyl hydroperoxide, which has the formula

CH₃C (CH₃)₂OOH (III)

is stated in EP-A-0531031 to be desirably used as an oxidising agent to prevent degradation of the disulfide agents.

European Patent Application No. 95306587.7 describes modifications of the above methods in which a non-toxic cysteine sulfoxide of formula 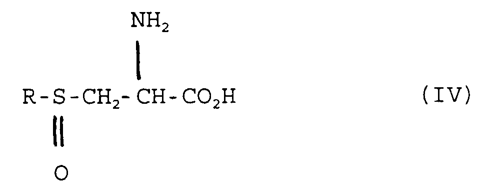 or a non-toxic salt thereof, in which R is a C₁₋₄ alkyl or a C₂₋₄ alkenyl group, is used in place of, or in addition to, the active agents of the prior art methods mentioned above to provide a therapeutic method (e.g. an anticancer therapy or a method for enhancing T-cell count) applicable *in vivo* to a patient or *in vitro* to a transfusable body fluid or transplantable body part. The active agent(s) is/are administered to the patient, body fluid or body part in an effective amount and, while present, there is administered to the patient, body fluid or body part an effective dose of microwave electromagnetic radiation of frequency in the range of about 400-450 MHz.

T-butyl hydroperoxide (III) is stated in European Patent Application No. 95306587.7 to be desirably used as an oxidising agent preceding the administration of the active agent(s).

The present invention is based on my surprising finding that the above methods have the further effect, hitherto unappreciated, of reducing the concentration of intracellular pathogenic viral and/or prion particles in body tissue and fluids.

The invention may therefore be stated to provide a therapeutic method for reducing the concentration of pathogenic viral and/or prion particles in body tissue and fluids, applicable *in vivo* to a patient or *in vitro* to a transfusable body fluid or transplantable body part, which comprises administering to the patient, body fluid or body part an effective amount of a non-toxic active agent selected from the non-toxic organic disulfides cystine and oxidised glutathione, the non-toxic cysteine sulfoxides of the general formula 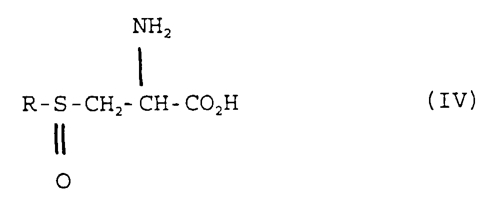 (in which R is a C₁₋₄ alkyl or a C₂₋₄ alkenyl group), and non-toxic salts thereof, and while said non-toxic agent is present administering to the patient, body fluid or body part an effective dose of microwave electromagnetic radiation of frequency in the range of about 400-450 MHz.

According to a further aspect, the invention provides the use of a non-toxic agent selected from the non-toxic organic disulfides cystine and oxidised glutathione, the non-toxic cysteine sulfoxides of formula IV, and non-toxic salts thereof, in the preparation of a composition or compositions for use in a therapeutic method according to the invention as described above.

The expression "non-toxic" used herein refers to an acceptably low level of toxicity when a compound is present at an effective amount, and not necessarily to a complete absence of toxic effects. In particular, compounds which have only a temporary toxic effect and do no significant permanent harm, or for which any toxic effects can readily be counteracted by the administration of a non-toxic "antidote" agent, will be referred to and understood as "non-toxic" herein.

The expression "pathogenic viral and/or prion particles" used herein includes all disease-causing infective particulate agents having a size smaller than bacterial pathogens, which are growing exponentially *in vivo* or in culture media *(in vitro).* Prion particles are generally smaller than viral particles.

Virus- and prion-propagated diseases treatable by the method of the invention include acquired immunodeficiency syndrome (AIDS), bovine spongiform encephalopathy (BSE or "Mad Cow" disease), scrapie, hepatitis, disseminated ("multiple") sclerosis (MS) and Creutzfeld Jacob disease (CJD). The corresponding viral and/or prion particles are destroyed when growing exponentially, by the method of the invention.

Thus, in preferred aspects, the invention provides a therapeutic method for reducing the concentration of infective particulate agents selected from human immunodeficiency virus (HIV), BSE virus, scrapie virus, hepatitis virus, MS virus and CJD virus in body tissue and fluids, and the use of the active agent(s) as defined above in the preparation of a composition or compositions for use in such a method.

If desired, more than one active agent as defined above may be used simultaneously or sequentially according to the invention. An oxidising agent, preferably t-butyl hydroperoxide (III), is desirably administered separately before the active agent(s) to prevent or restrict degradation of the active agent(s).

The compounds of formula IV and their non-toxic salts, as well as cystine, oxidised glutathione and their non-toxic salts, are non-toxic in doses exceeding 100 mg per kilogram body weight.

In the compounds of formula IV, R is optionally substituted and is preferably methyl, ethyl, propyl, butyl or allyl. An optically active form of the compound, or alternatively a racemic mixture, may be used. The laevorotatory (L) form is preferred. The non-toxic salts are preferably non-toxic acid or base addition salts, for example addition salts of strong acids or bases.

Specifically, the most preferred active agents of formula IV are S-methyl-L-cysteine sulfoxide, S-ethyl-L-cysteine sulfoxide, S-propyl-L-cysteine sulfoxide, S-butyl-L-cysteine sulfoxide and mixtures thereof. A mixture of the methyl, ethyl and butyl sulfoxides (e.g. in a 3:2:1 weight ratio) has been found to be the particularly effective.

An optically active form of cystine or oxidised glutathione, or alternatively an optically inactive (internally compensated) form or a racemic mixture, may be used. The laevorotatory form is preferred by reason of its availability.

In the optimum *in vivo* method the active agent(s) is/are used in association with the oxidising agent t-butyl hydroperoxide (III), to prevent reduction of the disulfides or sulfoxides *in vivo,* particularly in the liver. A stock solution of 70% t-butyl hydroperoxide in water, diluted up to 60-fold (by volume) in normal (0.9%) saline, e.g. around 20-fold, may conveniently be used. It is preferred that the oxidising agent be intravenously administered in the *in vivo* methods, prior to and separately from the sulfoxide active agents. It has been found that 60 ml of the above saline solution of oxidising agent can safely and effectively be administered over a period of about 5 minutes. The amount, formulation and intravenous administration route for the oxidising agent can readily be selected by a worker of ordinary skill in this art.

Cystine (and non-toxic salts thereof) and oxidised glutathione (and non-toxic salts thereof) may suitably be used in association with one or more alternative oxidising agent, for example hydrogen peroxide, to prevent degradation of the disulfides to corresponding thiols at least prior to administration. One ml of 10% hydrogen peroxide aqueous solution per gram of the disulfide to be administered is typically adequate, although the amount may be varied according to the particular requirement of the active agents used, in a manner which will be well within the capacity of a worker of ordinary skill in this art.

The composition form(s) by which the active agent(s) and any oxidising agent(s) is or are administered may take any suitable form and employ any suitable conventional pharmaceutical carrier(s) or excipient(s). The active agents should be administered parenterally for the *in vivo* methods. The composition form is preferably an aqueous solution or suspension suitable for intravenous drip, infusion or injection. Intravenous drip, infusion or injection should be used as the administration route by virtue of its rapid delivery of the active agent(s) to the bloodstream or to a cancer site with minimal degradation of the active agent(s). In the *in vitro* aspects of the invention, the active agent (s) are suitably contacted with the body fluid or body part to be treated as an aqueous solution or suspension.

The solution or other composition form may if desired include additional components such as salts (e.g. sodium chloride), solubilising and/or suspending agents and the like. As mentioned above, oxidants such as hydrogen peroxide or organic hydroperoxides may be used.

A suitable composition for use in a method of therapy according to the invention thus comprises an aqueous solution of one or more non-toxic cysteine sulfoxide of general formula IV, e.g. methyl, ethyl, propyl and/or butyl cysteine sulfoxide (or a non-toxic salt thereof) at a concentration of up to approximately 100 g/l (e.g. approximately 90-110 g/l), together with cystine and/or oxidised glutathione and/or a non-toxic salt thereof at a concentration of up to approximately 40 g/l (e.g. approximately 30-50 g/l).

The composition may suitably take the form of a kit of two or more compositions containing the agents, selected from compounds of formulae I, II, III and IV and non-toxic salts thereof, for rapidly sequential administration in a method of therapy according to the invention, a first composition comprising an aqueous solution containing a first agent selected from the agents as defined above, and a second composition comprising an aqueous solution containing a second agent selected from the agents as defined above, with the proviso that the composition to be administered first in *in vivo* therapy includes the compound of formula III; the said first and second compositions being optionally together with further composition(s) and instructional literature for the therapy.

The compositions are prepared by standard mixing techniques that will be readily apparent to those skilled in this art. In the case of sparingly soluble active agents a suitable solubilising agent should first be dissolved in the aqueous medium.

Aqueous solutions of the active agent(s) of formula IV and their salts at concentrations of up to around 100 grams per litre are convenient, enabling a daily injection volume of 50-60 ml to deliver around 5 grams of the desired sulfoxide intravenously. In the case of a mixture of active agents of formula IV, a total weight of up to around 15 grams (e.g. 12 grams) in a volume of around 60 ml is convenient.

If cystine is present, an aqueous solution of L-cystine at a concentration of approximately 40 grams per litre is convenient, enabling a daily injection volume about 50 to 60 ml, delivering a daily dose of up to approximately 2 g. To assist the passing of L-cystine into solution, a solubilising agent such as N-methyl-D-glucamine is also conveniently present in the solution. A small amount of an oxidant such as hydrogen peroxide may also be present to prevent the L-cystine from degrading to its thiol cysteine prior to administration. To avoid any risk of nausea, L-cystine should be injected slowly.

If oxidised glutathione is present, an aqueous solution of oxidised glutathione at a concentration of approximately 40 grams per litre is convenient, enabling a daily injection volume of about 50 to 60 ml, to deliver approximately 2 g of oxidised glutathione.

After administration (to the patient, or *in vitro* to the body fluid or part) of the active agent(s) according to the invention and any required oxidising agent(s), the microwave radiation should normally be begun to be administered between about 1 and 5 minutes later.

To administer *in vivo* the microwave radiation, preferably over the entire body of the patient, the patient is brought close to a suitable number of microwave antennae (e.g. 3 or 4 antennae arranged to encircle the patient's body) and the antennae energised to transmit UHF microwave radiation. Normally the patient may conveniently be moved under the antennae.

The total antennae power should preferably be less than about 4 kW (e.g. adjustable between about 1 and about 2 kW) in order to remain tolerable to the average patient. Patients may preferably be treated in two or more sessions, preferably three sessions, per day at daily intervals or every other or every third day for a treatment period of up to about four weeks.

Preferably, each session involves administration of the oxidising and active agent (s) followed over the subsequent 30 minutes by from 1 to about 5 exposures (e.g. three exposures) to the microwave radiation, each exposure to the radiation lasting for about 5 to 10 minutes, with a 2 to 5 minute rest period between exposures. The total length of treatment on each session day will be dependent on the length of time over which the agent(s) remain in the patient's body without substantial loss, and the length of rests between exposures which the patient requires. For optimum results each session should be terminated within 30 minutes of the first agent being administered (by intravenous injection). Small bodies (e.g. children) and adults can normally tolerate the same radiation power of about 1.6 to 2 kW per session.

Therapy using injections of t-butyl hydroperoxide (III) followed by compound IV and optionally cystine (I) and/or oxidised glutathione (II), followed 1 to 5 minutes later by suitable microwave radiation, produces immediate improvement in the concentration of pathogenic viral and/or prion particles, at optimum after approximately 5 to 25 days later.

As mentioned above, the microwave radiation should be in the frequency range of about 400-450 MHz. More preferably, the frequency should be in the range of about 425-450 MHz, most preferably 432-436 MHz (e.g. about 434 MHz).

Each antenna may consist of a single circular turn approximately 19 cm in diameter (the diameter being chosen in order that it resonates at the frequency of the transmitter), the plane of the circular antenna being held substantially tangential to the curvature of the patient's body, and within about 10 cm from the patient's skin. Such a circular antenna may emit predominantly H-wave microwave radiation of wavelength between about 65 and 75 cm (most preferably about 69 cm) towards the patient's body.

While the presence of E-wave radiation generated by dipole antennae (folded or of other configuration) will not necessarily hinder the efficacy of the radiation, it can lead to greater heating of the patient's skin and a poorer depth dose than a circular or loop antenna; such effects are undesirable and dipole antennae are therefore less preferred.

Each antenna is suitably connected to a UHF generator by a coaxial cable. Dipole antennae require circulators to prevent adverse interaction between antennae. Circular or loop antennae, which can be tuned accurately to resonance at the frequency of the generator, do not require circulators. Each generator may if desired be set at a slightly different frequency at or around a central frequency in the range already mentioned (preferably about 434 MHz). Each antenna is preferably energised at about 400 W.

All equipment within about 2 metres of the antennae should be of non-metallic materials such as wood or plastic, including for example the patient support table.

Administration of the microwave radiation in the *in vitro* method of the invention may be performed by any appropriate microwave emitting device, as will be readily understood by a worker of ordinary skill in this art, provided that steps are taken to prevent overheating of the material being treated.

Tests have yielded the following data, which are included purely for ease of understanding of the present invention, and not for limitation of the scope of the invention:

### EXAMPLE

### Compositions and administration volumes

Aqueous solutions of t-butyl hydroperoxide are prepared by dissolving 80 ml of a 70% aqueous solution of t-butyl hydroperoxide in 1 litre of normal (0.9%) saline. 15-30 ml of this solution is given intravenously over a period of up to about 15 minutes.

Aqueous solutions of methyl, ethyl, propyl and butyl cysteine sulfoxide are prepared by dissolving 100 gram of the respective sulfoxide in 1 litre of normal (0.9%) saline. 30-60 ml of this solution (delivering an approximate 2 to 6 gram dose of the sulfoxide) is given intravenously.

An aqueous solution of L-cystine is prepared by first dissolving 120 grams of N-methyl-D-glucamine in 1 litre of normal (0.9%) saline and the dissolving in 40 g of L-cystine. 50 to 60 ml of the solution (delivering an approximately 2 g dose of cystine) is given intravenously.

An aqueous solution of oxidised glutathione is prepared by dissolving 40 grams in 1 litre of normal (0.9%) saline. 50-60 ml of this solution (delivering an approximately 2 g dose of oxidised glutathione) is given intravenously.

### Microwave Therapy

The t-butyl hydroperoxide solution is administered by intravenous infusion over a period of up to about 15 minutes. This is then followed immediately by intravenous infusion of the sulfoxide, optionally with cystine and/or oxidised glutathione solutions, and then (within three minutes) Ultra High Frequency microwave radiation is commenced and is delivered over the next 20 - 30 minutes. 1 or 2 rest intervals of 2 to 3 minutes each are permitted during this application.

This can be repeated daily as often as is necessary to cause the desired reduction in the concentration of pathogenic viral and/or prion particles.

### Toxicity

No symptoms suggestive of any toxicity have been noted after the intravenous administration of t-butyl hydroperoxide, L-cystine, oxidised glutathione or methyl, ethyl, propyl, butyl or allyl cysteine sulfoxide. No immediate or late changes in blood pressure, renal or liver function, electrolytes or haematology have been seen from the method.

### Contra-indications

A prime contraindication is the presence of large collections of fluid in the pleural or peritoneal cavities. Static collections of fluid which contain high concentrations of salts have heat generated in them by the application of these magnetic fields. Patients with pleural or peritoneal effusions have blockage of the lymphatics draining these areas and also involvement of the pleural and peritoneal membranes. Unless these cavities are dry (less than 100 ml fluid when treated) the heating effect aggravates the irritation of the pleural and peritoneal cavities and the effusions become worse. Recommendations for treatment of such patients are as follows:
A: Peritoneal effusions. A Le Veen shunt or equivalent should be inserted prior to treatment and must be working so that the peritoneal cavity is kept dry. This drains the ascitic fluid back into the circulation.
B: Pleural effusions. A similar shunt can be used but is much less satisfactory. Pleural paracentesis to drain the cavity completely together with introduction of some agent causing sclerosis of the pleural cavity which obliterates it is more effective. The introduction of talc, tetracycline or another sclerosing agent appears the best way of preventing failure in the treatment.

Another contraindication to this treatment is where cancer of the stomach or bowels is causing partial obstruction to the gut. Treatment can cause breakdown of some of the cancer with a perforation of the bowel above the obstruction. All patients with bowel cancer should be fully assessed by a surgeon and if there is any evidence of obstruction the primary cancer should be removed or exteriorised by colostomy or some other similar procedure. This avoids the likelihood of perforation which can be lethal.

The following Case History is presented to show, without limitation, a clinical application of the method of the invention.

### CASE HISTORY

PH (a male): Diagnosed HIV at age 42. T4 level two years later was 300. Treated two months later according to the invention. T4 count varying between 500 and 700 seven months later. Retreated according to the invention over a 13 day period, commencing 18 months later. T4 1250 ("normal" is greater than 700). His T4-T8 ratio has returned to normal. His thrombocytopenia of 60,000 per mm³ has returned to 150,000 which is within normal limits (150,000 to 500,000). All his other haematological signs are normal. His secondary thrush infection of the throat has disappeared. He is no longer taking antibiotics. He has recently been tested with the 24VLP antigen and has a normal antigen response to all tests.

Measurements of his blood HIV-RNA (viral ribonucleic acid) using a Roche PCR (polymerase chain reaction) viral particle counter have shown a zero detectable level of HIV particles in the patient's blood on two occasions two months apart, following his treatment according to the invention.

## Claims

1. Use of a non-toxic active agent selected from the non-toxic organic disulfides cystine and oxidised glutathione, the non-toxic cysteine sulfoxides of the general formula 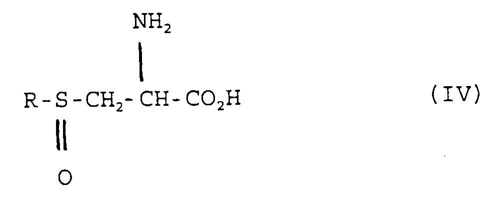 (in which R is a C₁₋₄ alkyl or a C₂₋₄ alkenyl group), and non-toxic salts thereof, in the preparation of a composition or compositions for use in a therapeutic method for reducing the concentration of pathogenic viral and/or prion particles in body tissue and fluids, applicable *in vivo* to a patient or *in vitro* to a transfusable body fluid or transplantable body part, which comprises administering to the patient, body fluid or body part an effective amount of the said non-toxic agent and while said non-toxic agent is present administering to the patient, body fluid or body part an effective dose of microwave electromagnetic radiation of frequency in the range of about 400-450 MHz.

2. A use according to claim 1, wherein the cysteine sulfoxide is selected from the group consisting of S-methyl-L-cysteine sulfoxide, S-ethyl-L-cysteine sulfoxide, S-propyl-L-cysteine sulfoxide, S-butyl-L-cysteine sulfoxide and mixtures thereof.

3. A use according to claim 2, wherein a mixture of S-methyl-L-cysteine sulfoxide, S-ethyl-L-cysteine sulfoxide and S-butyl-L-cysteine sulfoxide is used in a 3:2:1 weight ratio.

4. A use according to any of claims 1 to 3, wherein in the therapeutic method more than one active agent is administered simultaneously or sequentially.

5. A use according to any one of claims 1 to 4, wherein an oxidising agent is additionally present in the composition or at least one of the compositions, to prevent or restrict degradation of the active agent(s).

6. A use according to claim 5, wherein the oxidising agent is t-butyl hydroperoxide.

7. A use according to any one of claims 1 to 6, wherein the microwave electromagnetic radiation is administered at a frequency in the range of about 432-436 MHz.

8. A use according to any one of claims 1 to 7, wherein the viral and/or prion particles are infective particulate agents selected from human immunodeficiency virus (HIV), bovine spongiform encephalopathy (BSE) agent, scrapie agent, hepatitis agent, disseminated sclerosis (MS) agent and Creutzfeld Jacob disease (CJD) agent.

9. An *in vitro* method for reducing the concentration of pathogenic viral and/or prion particles, applied to a transfusable body fluid or a transplantable body part, which comprises administering to the body fluid or body part an effective amount of a non-toxic active agent selected from the non-toxic organic disulfides cystine and oxidised glutathione, the non-toxic cysteine sulfoxides of the general formula 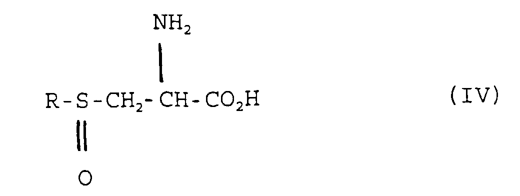 (in which R is a C₁₋₄ alkyl or a C₂₋₄ alkenyl group), and non-toxic salts thereof, and while said non-toxic agent is present at the body fluid or body part administering thereto an effective dose of microwave electromagnetic radiation of frequency in the range of about 400-450 MHz.
